Europäisches Patentamt

European Patent Office

Office européen des brevets

(11) Veröffentlichungsnummer: **0 045 917**
**B1**

(12) # EUROPÄISCHE PATENTSCHRIFT

(45) Veröffentlichungstag der Patentschrift:
14.05.86

(21) Anmeldenummer: 81106036.7

(22) Anmeldetag: 31.07.81

(51) Int. Cl.⁴: **A 61 B· 5/10**

(54) **Fingerabdrucksensor zum Erzeugen eines elektrischen Signals.**

(30) Priorität: **11.08.80 US 176701**

(43) Veröffentlichungstag der Anmeldung:
**17.02.82 Patentblatt 82/7**

(45) Bekanntmachung des Hinweises auf die Patenterteilung:
**14.05.86 Patentblatt 86/20**

(84) Benannte Vertragsstaaten:
**AT BE CH DE FR GB IT LI NL SE**

(56) Entgegenhaltungen:
**FR - A - 2 407 530**
**GB - A - 1 185 910**
**US - A - 3 882 462**

**IBM TECHNICAL DISCLOSURE BULLETIN, Band 16, Nr. 11, April 1974, Seiten 3572-3573, New York, U.S.A., D.T. FOLLETTE et al.: "Direct optical input system for fingerprint verification"**
**ABC DER OPTIK v. Karl Mütze, 1972, Seiten 770 und 771**

(73) Patentinhaber: **Siemens Aktiengesellschaft, Berlin und München Wittelsbacherplatz 2, D-8000 München 2 (DE)**

(72) Erfinder: **Rüll, Hartwig, Dr., Balduin-Helm-Strasse 39, D-8080 Fürstenfeldbruck (DE)**
Erfinder: **Devinney, Edward J., 100 Union Avenue, Delanco, New Jersey 08075 (US)**
Erfinder: **Chiu, Ming-Yee, Dr., 78 Boothby Drive, Mt. Laurel, New Jersey 08054 (US)**

## Beschreibung

Die Erfindung bezieht sich auf einen Fingerabdrucksensor zum Erzeugen eines elektrischen Signals nach dem Oberbegriff des Patentanspruchs 1.

Systeme zur Identifizierung eines durch Andrücken eines Fingers auf eine Kontaktfläche erzeugten Fingerabdrucks sind bekannt.

Beispielsweise geht aus der US-A-4 053 228 ein Fingerabdruckapparat hervor, der eine transparente Glasplatte aufweist, die als Kontaktfläche oder Fingerabdruckleser dient. Ein Fingerabdruck wird durch Andrücken des zu untersuchenden Fingers an die Rückfläche der Glasplatte und durch Stillhalten des Fingers darauf in einer vorbestimmten Position erzeugt. Der Fingerabdruck wird von einem die Vorderfläche der Glasplatte durchstrahlenden Lichtstrahl abgefragt. Der abfragende Strahl wird an der Rückfläche teilweise reflektiert, wodurch ein Signalstrahl erzeugt wird, der die Fingerabdruckinformation trägt. Der reflektierte Signalstrahl wird dann mit einem Hologramm des gleichen Fingerabdrucks korreliert. um dadurch die Identifizierung der Person zu erhalten.

Aus der US-A-4 120 585 geht ein anderes System zur Identifizierung eines Fingerabdrucks hervor. Dieses System weist als Fingerabdrucksensor ein nachgiebiges optisches Prisma auf. Die Basis des Prismas wird vom Finger der zu untersuchenden Person berührt. Das nachgiebige Prisma verformt sich unter dem ausgeübten Druck. Es reflektiert teilweise einen abtastenden Lichtstrahl in Richtung einer fotoempfindlichen Vorrichtung, die aktiviert wird. Die fotoempfindliche Vorrichtung aktiviert ihrerseits weitere optische Komponenten des Systems zur Identifizierung eines Fingerabdrucks. Ein Fingerabdruckleser wird auf das Muster der Erhöhungen und Vertiefungen des Fingerabdrucks einer zu identifizierenden Person hin überprüft. Es sei darauf hingewiesen, dass bei diesem bekannten System die Prismenfläche als Ganzes verformt oder verbogen wird. Der Finger drückt kein Muster in die Fläche ein, so dass auch keine Konfiguration des topografischen Reliefs des Fingers erhalten wird.

Die bekannten Systeme zur Identifizierung eines Fingerabdrucks erfordern eine ziemlich aufwendige Technologie. Ein System zur Identifizierung eines Fingerabdrucks sollte jedoch leicht und billig zusammensetzbar sein und gleichzeitig sollte es eine hohe Bildqualität· des Fingerabdrucks liefern. Insbesondere sollte das Fingerabdruckbild frei von Verzerrungen oder anderen Bildfehlern sein.

Es ist daher die Aufgabe der Erfindung, einen Fingerabdrucksensor anzugeben, der leicht zusammengebaut werden kann und billig ist, der eine hohe Empfindlichkeit und Auflösung aufweist, der sehr zuverlässig arbeitet und der Fingerabdruckbilder hoher Qualität liefert, die in einem weiten Bereich frei von Verzerrungen oder anderen Bildfehlern sind.

Diese Aufgabe wird durch einen Fingerabdrucksensor der eingangs genannten Art gelöst, der die im kennzeichnenden Teil des Patentanspruchs 1 angegebenen Merkmale aufweist.

Durch diese Lösung ist ein Fingerabdrucksensor geschaffen, der die Fingerabdruckinformation eines berührenden Fingers in ein lesbares optisches Bild und von da in ein elektrisches Ausgangssignal umwandelt.

Das elektrische Ausgangssignal des Fingerabdrucksensors stellt die in dem Fingerabdruck enthaltene Information dar und kann zur weiteren Verarbeitung in einen Rechner eingelesen werden. Das Ausgangssignal ermöglicht auch die digitale Verarbeitung des Fingerabdruckbildes.

Es sind auch keine Laserabtasttechniken erforderlich.

Bevorzugte und vorteilhafte Ausführungsformen des Fingerabdrucksensors nach Anspruch 1 gehen aus den Unteransprüchen 2 bis 10 hervor.

Zusammenfassend kann gesagt werden, dass ein Fingerabdrucksensor zum Umwandeln der Fingerabdruckinformation eines berührenden Fingers in ein elektrisches Ausgangssignal geschaffen ist, der einen Kontaktkörper aufweist, welcher wenigstens teilweise aus einem transparenten und elastischen Material gebildet ist. Dieser Kontaktkörper weist eine Lichtempfangsfläche zur Lichtaufnahme und eine Kontaktfläche zur Aufnahme eines von dem zu untersuchenden Finger ausgeübten Kontaktdruckes auf. Die Kontaktfläche ist aus dem elastischen Material gebildet. Bevor eine Untersuchung eines Fingers gestartet wird, ist die Kontaktfläche glatt. Wenn jedoch ein Finger auf die Kontaktfläche gedrückt wird, ändert sich deren glatte Struktur entsprechend dem Fingerabdruckmuster, und zwar aufgrund der elastischen Eigenschaften des gewählten Materials. Nach Fortnahme des Fingers von der Kontaktfläche nimmt diese wieder ihre frühere Struktur ein, d.h. sie wird wieder eben und glatt.

Der Fingerabdrucksensor weist auch eine Lichtquelle auf. Die Lichtquelle sendet einen ersten Lichtstrahl in Richtung der Lichtempfangsfläche aus. Der Lichtstrahl durchstrahlt die Lichtempfangsfläche und erreicht die Kontaktfläche. Er trifft schräg auf die Lichtempfangsfläche auf, d.h. die Strahlrichtung und die Flächennormale der Lichtempfangsfläche schliessen einen Winkel ein. In Anwesenheit des andrückenden Fingers spiegelt die Kontaktfläche einen Lichtstrahl in der der Strahlrichtung des ersten Strahls entgegengesetzten Richtung, der die Lichtempfangsfläche ebenfalls durchstrahlt. Der gespiegelte Lichtstrahl ist mit dem Fingerabdruckmuster des Fingers moduliert.

Der Fingerabdrucksensor weist des weiteren ein optisches System und einen Fotodetektor auf. Der Fotodetektor weist einen lichtempfindlichen Bereich zur Messung der auf den Bereich auftreffenden Lichtverteilung auf. Das optische System führt das von der Kontaktfläche reflektierte und modulierte Licht dem lichtempfindlichen Bereich des Fotodetektors zu.

Im optischen Strahlengang zwischen dem Finger und dem lichtempfindlichen Bereich ist eine Blende vorgesehen, die zum Ausblenden eines Teiles des reflektierten Lichts dient. Insbesondere kann diese Blende ein scharfer, schneidenförmiger Rand oder eine Messerschneide sein, die das Bild der Lichtquelle ausblendet und die es ermöglicht, dass das vom Finger gespiegelte Licht zum lichtempfindlichen Bereich gelangt. Der Fingerabdrucksensor weist schliesslich auch einen Ausgang auf, der dem Fotodetektor zugeordnet ist und aus dem elektrische Ausgangssignal entnommen werden kann.

Das elastische Material, welches die Kontaktfläche des Kontaktkörpers bildet, kann ein Polymer sein. Insbesondere hat sich Silikonkautschuk oder -gummi, ein Elastomer, als brauchbar erwiesen.

In einer bevorzugten Ausführungsform weist der Kontaktkörper eine erste und zweite planare Seite oder Seitenfläche auf. Beide Seiten sind nicht exakt parallel zueinander angeordnet, sondern schliessen einen kleinen spitzen Winkel oder Keilwinkel $\gamma$ zwischen ihnen ein. Dieser Winkel kann kleiner als 5°, insbesondere kleiner als 3°, sein. Die erste dieser sich gegenüberliegenden und voneinander abgewandten Seiten oder Seitenflächen bildet die Lichtempfangsfläche und die zweite Seite die Kontaktfläche, die den Kontaktdruck des Fingers aufnimmt. In dieser Ausführungsform kann der Kontaktkörper ganz oder teilweise aus dem transparenten und elastischen Material gebildet sein. In einer bevorzugten Version ist eine Trägerplatte an eine aus dem elastischen und transparenten Material gebildete Sensorplatte angebracht. Die Trägerplatte kann aus Glas bestehen. Sie liefert einen ausreichenden Widerstand, wenn ein Finger auf die Kontaktfläche gedrückt wird. Da ein keilförmiger Kontaktkörper benutzt wird, wird von der Lichtempfangsfläche reflektiertes Licht relativ zu dem vom Fingerabdruck abgestrahlten Licht seitwärts verschoben, insbesondere wenn Licht fokussierende Mittel benutzt werden. Deshalb kann von der Lichtempfangsfläche reflektiertes Licht, das keine Fingerabdruckinformation enthält, durch eine Schneide oder eine entsprechende Blende ausgeblendet werden.

Ausführungsformen der Erfindung sind in den Figuren dargestellt und werden in der folgenden Beschreibung näher erläutert. Von den Figuren zeigen:

Figur 1 eine Ausführungsform eines Fingerabdrucksensors, der eine Sensorplatte und eine keilförmige Trägerplatte aufweist,

Figur 2 einen Querschnitt durch den Bereich zwischen einem Finger und der Sensorplatte,

Figur 3 einen keilförmigen Kontaktkörper, der von einem zu untersuchenden Finger berührt wird,

Figur 4 eine Ausführungsform eines Fingerabdrucksensors, der eine Sensorplatte und eine mit einem nicht reflektierenden Medium beschichtete Trägerplatte aufweist, und

Figur 5 eine Sensorplatte als Kontaktkörper, die mit einem nicht reflektierenden Medium beschichtet ist und die von einem zu untersuchenden Finger berührt wird.

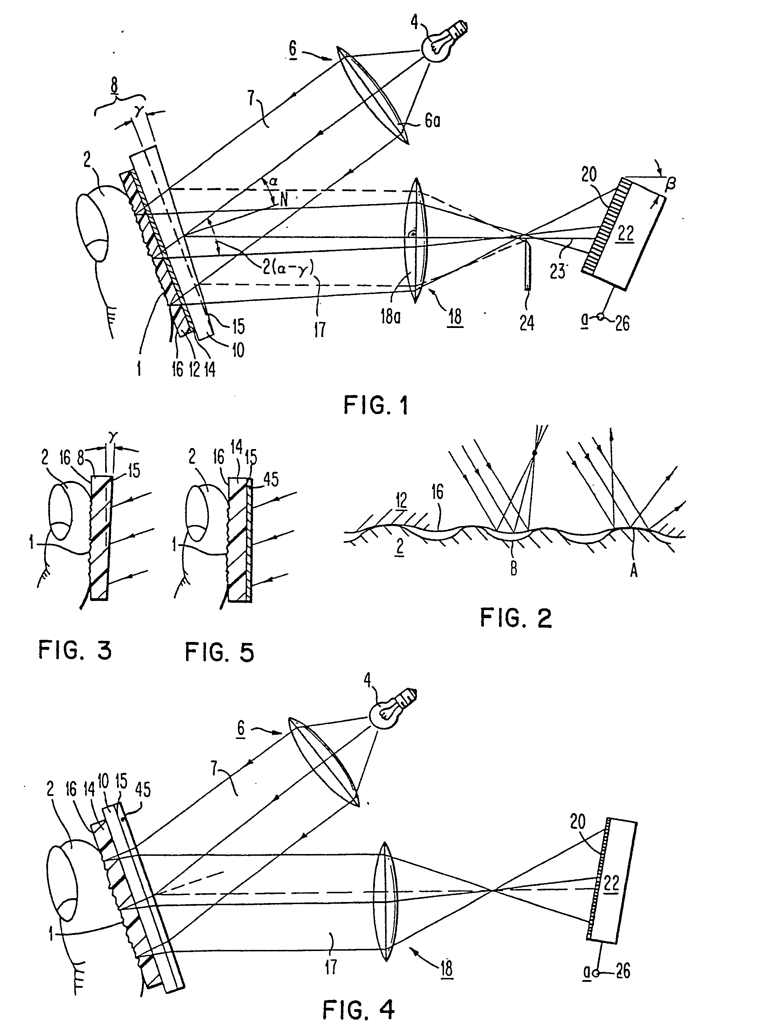

In der Figur 1 ist ein Fingerabdrucksensor zum Erzeugen eines dem topografischen Relief, Muster oder Abdruck 1 eines zu untersuchenden Fingers 2 entsprechenden elektrischen Signals a dargestellt.

Der Fingerabdrucksensor weist eine Lichtquelle 4 auf, die einen zum Abtasten des Fingerabdrucks 1 dienenden Lichtstrahl aussendet. Die Lichtquelle 4 kann eine Weisslichtquelle sein, beispielsweise eine reguläre Glühbirne. In der Ausführungsform gemäss Figur 1 ist die Lichtquelle 4 eine kleine Glühbirne. Die Lichtquelle 4 kann auch eine lichtemittierende Diode (LED) oder ein Halbleiterlaser sein, der Licht einer spezifischen Wellenlänge aussendet. Die Wellenlänge des Lichts kann im Infrarotbereich des Spektrums liegen. Es ist jedoch einer der Vorteile der Erfindung, dass eine Lichtquelle 4 verwendet werden kann, die ein breites Spektrum aussendet.

Das Licht aus der Lichtquelle 4 wird von einem ersten optischen System 6, das eine Kollimatorlinse 6a aufweist, gebündelt. Das optische System 6 richtet einen parallelen Lichtstrahl 7 in Richtung eines transparenten Kontaktkörpers 8. Wenn eine kleine Lichtquelle 4 benutzt wird, die weit entfernt angeordnet ist, kann das erste optische System 6 weggelassen werden.

In der Figur 1 ist der Kontaktkörper 8 als eine keilförmige Plattenkonstruktion ausgebildet. Diese weist eine Trägerplatte 10 und eine flache Kontakt- oder Sensorplatte 12 auf. Die Trägerplatte 10 weist zwei sich gegenüberliegende Seiten auf, die einen kleinen spitzen Winkel oder Keilwinkel $\gamma$ einschliessen. Die Sensorplatte 12 weist sich gegenüberliegende Seiten auf, die parallel zueinander sind. «Kleiner spitzer Winkel» soll bedeuten, dass der Winkel kleiner als 20° ist. Der Winkel $\gamma$ sollte vorzugsweise kleiner als 5° sein. In Abhängigkeit von der Geometrie kann er kleiner als 3° sein. Zwischen den beiden Platten 10 und 12 ist ein optisches Anpassungsmedium 14 angeordnet. Das Anpassungsmedium 14 besteht aus einer Substanz, die den Brechungsindex der Trägerplatte 10 an den Brechungsindex der benachbarten Sensorplatte 12 anpasst.

Wie erwähnt, weist der Kontaktkörper 8 eine keilförmige Trägerplatte 10 und eine flache oder plane Sensorplatte 12 auf. Die Trägerplatte 10 weist eine innere und eine äussere planare Seite auf. Die äussere oder rechte Seite bildet eine Lichtempfangsfläche 15. Die Flächennormale der Lichtempfangsfläche 15 ist mit N bezeichnet.

Die Sensorplatte 12 ist aus transparentem, elastischem Material gebildet. Dieses elastische Material kann aus einem Elastomer, beispielsweise einem Polymer, bestehen. Vorzugsweise besteht das Material der Sensorplatte 12 aus Silikonkautschuk oder -gummi. Die Sensorplatte 12 weist eine innere und eine äussere planare Seite auf. Beide Seiten sind parallel zueinander angeordnet. Die äussere oder linke Seite bildet eine Kontaktfläche 16, auf die mittels des Fingers 2 ein Kontaktdruck ausgeübt wird. Wenn der Finger 2 die Sensorplatte 12 nicht berührt, ist die Kontaktfläche 16 glatt und eben. Wenn jedoch der Finger 2 auf die Kontaktfläche 16 gedrückt wird, ändert sich die Struktur dieser Fläche. Die Kontaktfläche 16 enthält jetzt ein Muster, das dem topografischen Relief der Erhöhungen und Vertiefungen des Fingerabdrucks entspricht.

Die Trägerplatte 10 gibt dem Kontaktkörper 8 eine Steifigkeit und Festigkeit, die erforderlich ist, wenn der Finger 2 auf die Kontaktfläche 16 gepresst wird. Die Trägerplatte 10 kann aus transparentem Kunststoff gebildet sein. Vorzugsweise ist sie aus Glas gebildet. Vorzugsweise wird ein Material gewählt, dessen Brechungsindex annähernd gleich dem Brechungsindex des elastischen Materials ist.

Der Lichtstrahl 7 aus der Lichtquelle 4 trifft schräg auf die Lichtempfangsfläche 15. Der Einfallswinkel bezüglich der Flächennormalen N ist mit α bezeichnet. Der Lichtstrahl 7 durchstrahlt die Lichtempfangsfläche 15 und tritt in die Trägerplatte 10 ein. Dann durchstrahlt er das Anpassungsmedium 14. Daraufhin tritt er in die Sensorplatte 12 ein und trifft schliesslich auf die Kontaktfläche 16 der Sensorplatte 12. Wenn die Kontaktfläche 16 von der Haut des Fingers 2 berührt wird, findet eine teilweise Spiegelreflexion statt.

Der von dem Kontaktkörper 8 reflektierte Lichtstrahl 17 ist teilweise in durchgezogenen Linien und teilweise in gestrichelten Linien dargestellt. Ein durch die gestrichelten Linien dargestellter Anteil des Lichtstrahls 17 wird von der Empfangsfläche 15 reflektiert. Dieser Anteil enthält keine Fingerabdruckinformation. Der durch die durchgezogenen Linien dargestellte Anteil wird vom Fingerabdruck 1 reflektiert. Dieser Anteil ist mit der topologischen Struktur des Fingers 2 räumlich moduliert. Der reflektierte Strahl 17 trägt deshalb insgesamt die Information über den Fingerabdruck. Er wird zur rechten Seite des Fingerabdrucksensors reflektiert, d. h. zu der Seite, auf der die Lichtquelle 4 angeordnet ist.

Der Lichtstrahl 17 durchstrahlt ein zweites optisches System 18. Gemäss Figur 1 weist das zweite optische System 18 eine Linse 18a oder das Linsensystem einer Kamera, das eine grosse Öffnung aufweist, auf.

Der reflektierte Lichtstrahl 17 trifft schliesslich teilweise auf den lichtempfindlichen Bereich 20 eines Fotodetektors 22. Der Fotodetektor 22 misst die Verteilung des auf seinen lichtempfindlichen Bereich 20 auftreffenden Lichts.

Der lichtempfindliche Bereich 20 kann aus einer Anordnung einzelner fotoempfindlicher Elemente bestehen. Die Ausgangssignale dieser Elemente werden in eine digitale Bildverarbeitungseinrichtung eingespeist, die in den Fotodetektor 22 eingebaut ist. Aus der Ausgabe 26 des Fotodetektors wird das elektrische Ausgangssignal a abgeleitet.

Der Fotodetektor 22 ist in bezug auf die Abbildungslinse 18a des zweiten optischen Systems 18 so angeordnet, dass das Bild des Fingerabdrucks scharf auf den lichtempfindlichen Bereich 20 abgebildet oder fokussiert wird. Zu diesem Zweck ist der Fotodetektor 22 gegen die optische Achse 23 des zweiten optischen Systems um einen Winkel β verkippt, wobei die Verkippung um eine zur optischen Achse 23 senkrechte Achse erfolgt ist.

Zwischen der Abbildungslinse 18a und dem lichtempfindlichen Bereich 20 des Fotodetektors 22 ist eine Ausblendvorrichtung 24 angeordnet, die eine scharfe messerartige Kante, eine Rasierklinge oder eine kleine Scheibe sein kann, und die für einen guten Kontrast im Fingerabdruckbild sorgt. Die Ausblendvorrichtung oder Blende 24 ist so angeordnet, dass ihre Spitze den reflektierten Lichtstrahl 17 teilweise ausblendet. Insbesondere ist sie so angeordnet, dass sie das Bild der Lichtquelle 4 ausblendet. Insbesondere ist sie auch so angeordnet, dass sie den von der Lichtempfangsfläche 15 direkt reflektierten und in gestrichelten Linien dargestellten Lichtanteil ausblendet, so dass dieser nicht zum lichtempfindlichen Bereich 20 gelangen kann. Wenn ein einen Parallelstrahl 7 erzeugendes optisches System 6 verwendet wird, sollte die Spitze der Blende 24 in der Brennebene der Abbildungslinse 18a angeordnet sein, in der das direkt reflektierte Licht fokussiert wird. Messerkanten oder -schneiden oder andere Raumfilter werden in der sogenannten Schlierenmethode (Siehe OPTICS, Febr. 1979, S. 478–481) verwendet.

Wie schon erwähnt, kann als transparentes Wandlermaterial für die Sensorplatte 12 ein Silikonpolymer oder -gummi verwendet werden. Als Anpassungsmedium 14 kann Silikonöl verwendet werden. Dieses Öl erzeugt einen guten mechanischen Kontakt und eine gute optische Zwischenschicht zwischen der Sensorplatte 12 und der Trägerplatte 10 aus Glas. Durch Verwendung von Silikonöl oder einem anderen Anpassungsmedium 14 kann Vielfachreflexion vermieden werden. Anstelle von Silikonöl kann auch ein optischer Kitt verwendet werden, der einen geeigneten Brechungsindex aufweist. Auch ein optischer Kleber mit den gleichen Eigenschaften kann zum direkten Verbinden der Sensorplatte 12 mit der Trägerplatte 10 aus Glas verwendet werden.

Bei einer nicht dargestellten anderen Ausführungsform kann das Material der Sensorplatte 12, beispielsweise ein Silikonpolymer, direkt die linke oder äussere Seite der Trägerplatte 10 bedecken. Bei dieser Ausführungsform ist ein Anpassungsmedium 14 nicht erforderlich.

Der Fotodetektor 22 kann aus einer Standard-Videokamera (Vidikonkamera) bestehen. Eine derartige Videokamera weist den Vorteil auf, dass sie eine Schnittstelle zu einem Mikrorechner bilden kann. Es ist jedoch auch möglich, eine im Handel erhältliche CCD-Matrix oder eine CID-Matrix als lichtempfindlichen Bereich 20 zu verwenden. Eine derartige Ausführungsform kann aus Gründen der Einfachheit und Zuverlässigkeit gewählt werden. Sie kann auch die Kosten und die Grösse des Fingerabdrucksensors reduzieren.

Es sei noch einmal darauf hingewiesen, dass die Lichtquelle 4 die Lichtempfangsfläche 15 unter einem Neigungswinkel α beleuchtet. Deshalb wird sowohl das von der Lichtempfangsfläche 15 (Grenzfläche Luft-Glas) als auch das vom Fingerabdruck 1 (Grenzfläche Elastomer-Luft) gespiegelte Licht von dem die grosse Apertur aufweisenden Kameraobjektiv 18a des zweiten optischen Systems 18 eingefangen. Die Reflexionen von den Gegenseiten der Platten 10 und 12 (Grenzfläche Glas-Elastomer) kann vernachlässigt werden, wenn die Brechungsindizes des Glases und des Elastomers annähernd gleich sind.

Das von der Lichtempfangsfläche 15 (Grenzfläche Luft-Glas) reflektierte Licht enthält keine Information über den Fingerabdruck und stellt eine Art Gleichlicht-Hintergrund dar, der dazu neigt, den Kontrast im Fingerabdruckbild herabzusetzen. Bei der hier beschriebenen Ausführungsform des Fingerabdrucksensors wird dieser Gleichlicht-Hintergrund durch die Verwendung einer Trägerplatte 10 eliminiert, die einen kleinen Keilwinkel γ aufweist, so dass das Bild der Lichtquelle 4 seitwärts verschoben und die Blende 24 ausgeblendet wird. Das von der Kontaktfläche 16 reflektierte Licht enthält die Fingerabdruckinformation und erzeugt ein Bild auf dem lichtempfindlichen Bereich 20. Das Ausgangssignal a entspricht diesem Bild und kann gespeichert und verarbeitet werden.

Gemäss Figur 2 wird die Kontaktfläche 16 entsprechend der Topologie der Haut verformt, wenn der Finger 2 auf die Kontaktfläche 16 gedrückt wird. In einem erhöhten Bereich A des Fingers 2 steht die Haut in Kontakt mit der Kontaktfläche 16 und es tritt Licht aus der Sensorplatte 12 aus. Das Licht wird von der rauhen Oberfläche der Haut des Fingers teilweise absorbiert und gestreut. In einem vertieften Bereich B der Haut ist die Kontaktfläche 16 jedoch eine gute optische Fläche, weil dort kein Kontakt zwischen der Kontaktfläche 16 und der rauhen Haut des Fingers stattfindet. Der vertiefte Bereich B spiegelt das Licht. Da das Material der Sensorplatte 12 flexibel ist, bildet die Kontaktfläche 16 lokal einen kleinen konkaven Brennspiegel. Es kann deshalb ein sehr starker Bildkontrast erhalten werden, wenn ein Brennpunkt der Abbildungslinse 18a in eine Ebene gelegt wird, die von der Ebene zwischen dem Finger 2 und der Sensorplatte 12 ein wenig beabstandet ist.

Der Abbildungsmassstab des Fingerabdrucks kann grob 1 : 1 sein. Eine Störung, die aufgrund einer schrägen Betrachtung des Fingers 2 auftreten kann, kann durch Verkippen des Fotodetektors 22 um einen Kippwinkel β = α + γ korrigiert werden. Eine derartige Verkippung bewirkt, dass der fotoempfindliche Bereich 20 in der Bildebene des Fingerabdrucks 1 liegt, auf die dieser scharf abgebildet wird.

Aus der Figur 3 geht hervor, dass ein keilförmiger Kontaktkörper 8 verwendet werden kann, der ganz aus dem erwähnten transparenten Material gebildet sein kann. Sowohl die Empfangsfläche 15 als auch die Kontaktfläche 16 sind dann aus diesem Material gebildet.

Aus den Figuren 4 und 5 geht hervor, dass das von der Lichtempfangsfläche 15 reflektierte Licht auch durch Aufbringen eines nicht reflektierenden Überzugs 45 auf die Lichtempfangsfläche 15 einer Trägerplatte 10 bzw. eines ganz aus dem transparenten Material gebildeten Kontaktkörpers 8 eliminiert werden kann. In den Figuren 4 und 5 wirkt der Überzug 45 als Blende.

In der Ausführungsform nach Figur 4 weisen die Platte 10 und 14 parallele Seiten auf. Die äussere Seite der Trägerplatte 10, welche die Lichtempfangsfläche 15 bildet, ist mit einem dünnen Film 45 aus nicht reflektierendem Material, nämlich der Vergütungsschicht auf dem Objektiv einer Fotokamera. überzogen. In der Ausführungsform nach Figur 5 ist der dünne Film 45 direkt auf die Lichtempfangsfläche 15 der Kontaktplatte 14 aufgebracht, weil eine Trägerplatte 10 nicht benutzt wird.

**Patentansprüche**

1. Fingerabdrucksensor zum Erzeugen eines dem Papillarmuster der Haut eines Fingers (2) entsprechenden elektrischen Ausgangssignals, mit einer auf einer Seite eines transparenten Kontaktkörpers (8) ausgebildeten elastische Kontaktfläche (16) zum Aufdrücken des Fingers (2), in der das jeweils aufgedrückte Papillarmuster durch elastische Verformung ein entsprechendes topografisches Relief (1) erzeugt, einer Lichtquelle (4), welche das aufgedrückte Papillarmuster mit einem der Kontaktfläche (16) durch den transparenten Kontaktkörper (8) über eine von der Kontaktfläche (16) abgekehrte Lichtempfangsfläche (15) des Kontaktkörpers (8) schräg zugeführten Lichtstrahl (7) beleuchtet, der von der Kontaktfläche (16) reflektiert wird, wobei der reflektierte Lichtstrahl (17) dem topographischen Relief entsprechend moduliert wird, und mit einer optischen Einrichtung (18), die den reflektierten Lichtstrahl einem lichtempfindlichen Bereich (29) einer Fotodetektoreinrichtung (22) zuführt, die ein der Intensität des reflektierten Lichtstrahls (17) entsprechendes elektrisches Ausgangssignal (a) erzeugt, dadurch gekennzeichnet,

1) dass die optische Einrichtung (18) aus einer Abbildungsoptik besteht, die das dem topographischen Relief entsprechende Lichtmuster scharf auf den lichtempfindlichen flächenhaften Bereich (20) der Fotodetektoreinrichtung (22) und vor diesem Bereich (20) die Lichtquelle (4) abbildet,

2) dass eine Blende (24) so angeordnet ist, dass sie das Bild der Lichtquelle (4) ausblendet, die das Bild des topografischen Reliefs (1) erzeugenden Lichtstrahlen dagegen durchlässt, und

3) dass die Fotodetektoreinrichtung (22) so ausgebildet ist, dass sie die Verteilung der auf den lichtempfindlichen Bereich (20) auftreffenden Lichtintensität erfasst und dadurch ein dem topografischen Relief (1) entsprechendes elektrisches Ausgangssignal (a) erzeugt, das zugleich dem aufgedrückten Papillarmuster entspricht.

2. Sensor nach Anspruch 1, dadurch gekennzeichnet, dass die Kontaktfläche (16) und die davon abgekehrte Lichtempfangsfläche (15) in einem spitzen Winkel (γ) zueinander angeordnet sind, der kleiner als 5° ist.

3. Sensor nach Anspruch 2, dadurch gekennzeichnet, dass der spitze Winkel (γ) kleiner als 3° ist.

4. Sensor nach einem der vorhergehenden Ansprüche, dadurch gekennzeichnet, dass die Lichtempfangsfläche (15) einen reflexionsmindernden Überzug aufweist.

5. Sensor nach einem der vorhergehenden Ansprüche, dadurch gekennzeichnet, dass der Kontaktkörper (8) eine aus elastischem Material gebildete Sensorplatte (12, 14) aufweist, die auf einer Seite einer transparenten Trägerplatte (10) aufgebracht ist, und die von der Trägerplatte (10) abgewandte Seite der Sensorplatte (12, 14) die Kontaktfläche (16) und die von der Sensorplatte (12, 14) abgewandte Seite der Trägerplatte (10) die Lichtempfangsfläche (15) bildet.

6. Sensor nach Anspruch 2 oder 3 und 5, dadurch gekennzeichnet, dass die Sensorplatte (12, 14) parallele Seiten und die Trägerplatte (10) im kleinen spitzen Winkel zueinander angeordnete Seiten aufweist.

7. Sensor nach Anspruch 5 oder 6, dadurch gekennzeichnet, dass die Trägerplatte (10) aus einer Glasplatte besteht.

8. Sensor nach Anspruch 5, 6 oder 7, dadurch gekennzeichnet, dass zwischen der Sensorplatte (12) und der Trägerplatte (10) ein optisches Anpassungsmedium (14) angeordnet ist, das sowohl mit der Sensorplatte (12) als auch mit der Trägerplatte (10) in Kontakt steht.

9. Sensor nach einem der Ansprüche 1 bis 3, dadurch gekennzeichnet, dass der Kontaktkörper (8) eine Sensorplatte (14) aufweist, die aus einem elastischem Material besteht und in einem kleinen spitzen Winkel zueinander angeordnete Seiten aufweist, von denen die eine die Lichtempfangsfläche (15) und die andere die Kontaktfläche (16) bildet.

10. Sensor nach einem der Ansprüche 1 bis 3, dadurch gekennzeichnet, dass der Kontaktkörper (8) aus einer aus elastischem Material gebildeten Sensorplatte (14) mit parallelen Seiten besteht, von denen eine die Lichtempfangsfläche (15) und die andere die Kontaktfläche (16) bildet.

**Claims**

1. A fingerprint sensor for delivering an electric output signal which corresponds to the papillary pattern of the skin of a finger (2), having an elastic contact surface (16) formed on one side of a transparent contact body (8) which serves for the impression of the finger (2), in which the respectively impressed papillary pattern produces a corresponding topographic relief (1) of a light source (4) by elastic deformation, which light source illuminates the impressed papillary pattern by means of a light beam (7) supplied obliquely to the contact surface (16) through the transparent contact body (8) via a light receiving surface (15) of the contact body (8) remote from the contact surface (16), which light beam is reflected off the contact surface (16) with the reflected light beam (17) modulated in accordance with the topographic relief, and having an optical device (18) which supplies the reflected light beam to a light-sensitive region (29) of a photo detector device (22) which delivers an electric output signal (a) corresponding to the intensity of the reflected light beam (17), characterised in

(1) that the optical device (18) consists of a focussing optical system which sharply focusses the light pattern corresponding to the topographic relief onto the light-sensitive areal region (20) of the photo detector device (22) and focusses the light source (4) before the region (20),

(2) that a diaphragm (24) is arranged in such a manner that it occludes the image of the light source (4) but allows the light beams producing the image of the topographic image to pass through, and

(3) that the photo detector device (22) is designed to be such that it detects the distribution of the light intensity incident upon the light-sensitive region (20) and thus produces an electric output signal (a) which corresponds to the topographic relief (1) and simultaneously corresponds to the impressed papillary pattern.

2. A sensor as claimed in Claim 1, characterised in that the contact surface (16) and the light receiving surface (15) which faces away therefrom, are arranged to one another at an acute angle (γ) which is smaller than 5°.

3. A sensor as claimed in Claim 2, characterised in that the acute angle (γ) is smaller than 3°.

4. A sensor as claimed in one of the preceding Claims, characterised in that the light receiving surface (15) has a coating which reduces the reflection.

5. A sensor as claimed in one of the preceding Claims, characterised in that the contact body (8) has a sensor plate (12, 14) made of an elastic material applied onto one side of a transparent carrier plate (10) and the side of the sensor plate (12, 14) remote from the carrier plate (10) forms the contact surface (16) and the side of the carrier plate (10), remote from the sensor plate (10), forms the light receiving surface (15).

6. A sensor as claimed in Claim 2 or 3 and 5, characterised in that the sensor plate (12, 14) has parallel sides and the carrier plate (10) has sides which are arranged at a small acute angle to one another.

7. A sensor as claimed in Claim 5 or 6, characterised in that the carrier plate (10) consists of a glass plate.

8. A sensor as claimed in Claim 5, 6 or 7, characterised in that between the sensor plate (12) and the carrier plate (10) an optical matching medium (14) is in contact both with the sensor plate (12) and with the carrier plate (10).

9. A sensor as claimed in one of the preceding Claims 1 to 3, characterised in that the contact body (8) has a sensor plate (14) which consists of an elastic material and has sides arranged at a small acute angle to one another, one of which forms the light receiving surface (15) and the other the contact surface (16).

10. A sensor as claimed in one of Claims 1 to 3, characterised in that the contact body (8) consists of a sensor plate (14) of an elastic material and has parallel sides, one of which forms the light receiving surface (15) and the other forms the contact surface (16).

## Revendications

1. Détecteur d'empreintes digitales servant à produire un signal de sortie électrique correspondant au modèle papillaire de la peau d'un doigt (2), et comportant une surface de contact élastique (16) ménagée sur une face d'un corps de contact transparent (8) et servant à l'application du doigt (2) sous pression et dans laquelle le modèle papillaire respectivement comprimé produit, sous l'effet d'une déformation élastique, un relief topographique correspondant (1), une source de lumière (4) qui éclaire le modèle papillaire comprimé, avec un faisceau lumineux (7) envoyé obliquement à travers le corps de contact transparent (8), par l'intermédiaire d'une surface réceptrice de la lumière (15), tournée à l'opposé de la surface de contact (16), du corps de contact (8) et qui est réfléchi par la surface de contact (16), le faisceau de lumière réfléchi (17) modulant de façon correspondante le relief topographique, et un dispositif optique (18) qui envoie le faisceau de lumière réfléchi dans une zone photosensible (29) d'un dispositif photodétecteur (22) qui produit un signal de sortie électrique (a) correspondant à l'intensité du faisceau de lumière réfléchi (16), caractérisé par le fait que

1) le dispositif optique (18) est constitué par un système optique de formation d'images, qui forme de façon précise le modèle lumineux correspondant au relief topographique sur la zone photosensible (20), d'une certaine étendue, du dispositif photodétecteur (22) et forme l'image de la source de lumière (4) en avant de cette zone (20),

2) qu'un diaphragme (24) est disposé de telle sorte qu'il bloque la lumière de la source de lumière (24), en laissant passer au contraire les rayons lumineux produisant l'image du relief topographique (1) et

3) que le dispositif photodétecteur (22) est agencé de telle sorte qu'il détecte la distribution de l'intensité lumineuse tombant sur la zone photosensible (20), et produit de ce fait un signal de sortie électrique (a) correspondant au relief topographique (1) et correspondant simultanément au modèle papillaire appliqué sous pression.

2. Détecteur suivant la revendication 1, caractérisé par le fait que la surface de contact (16) et la surface réceptrice de lumière (15), tournée à l'opposé de cette surface de contact, font entre elles un angle aigu ($\gamma$), qui est inférieur à 5°.

3. Détecteur suivant la revendication 3, caractérisé par le fait que l'angle aigu ($\gamma$) est inférieur à 3°.

4. Détecteur suivant l'une des revendications précédentes, caractérisé par le fait que la surface réceptrice de lumière (15) possède un revêtement réduisant les réflexions.

5. Dispositif suivant l'une des revendications précédentes, caractérisé par le fait que le corps de contact (8) comporte une plaque de détection (12, 14) qui est formée en un matériau élastique et qui est montée sur une face d'une plaque de support transparente (10), et que la face de la plaque de détection (12, 14), tournée à l'opposé de la plaque de support (10), forme la surface de contact (16) et que la face de la plaque de support (10), tournée à l'opposé de la plaque de détection (12, 14), constitue la surface réceptrice de lumière (15).

6. Détecteur suivant les revendications 2 ou 3 et 5, caractérisé par le fait que la plaque de détection (12, 14) comporte des faces parallèles et que la plaque de support (10) comporte des faces faisant entre elles un angle aigu faible.

7. Détecteur suivant les revendications 5 ou 6, caractérisé par le fait que la plaque de support (10) est constituée par une plaque de verre.

8. Détecteur suivant la revendication 5, 6 ou 7, caractérisé par le fait qu'entre la plaque de détection (12) et la plaque de support (10) se trouve disposé un milieu d'adaptation optique (14) qui est en contact aussi bien avec la plaque du détecteur (12) qu'avec la plaque de support (10).

9. Détecteur suivant l'une des revendications 1 à 3, caractérisé par le fait que le corps de contact (8) comporte une plaque de détection (14), qui est constituée en un matériau élastique et comporte des faces faisant entre elles un angle aigu et dont l'une forme la surface réceptrice de la lumière (15) et dont l'autre forme la surface de contact (16).

10. Détecteur suivant l'une des revendications 1 à 3, caractérisé par le fait que le corps de contact (8) est constitué par une plaque de détection (14) formée en un matériau élastique et comportant des faces parallèles, dont l'une forme la surface réceptrice de la lumière (15) et dont l'autre forme la surface de contact (16).

FIG. 1

FIG. 3    FIG. 5

FIG. 2

FIG. 4